# EUROPÄISCHE PATENTSCHRIFT

(11) **EP 3 544 656 B1**
(45) Veröffentlichungstag und Bekanntmachung des Hinweises auf die Patenterteilung: **11.02.2026**
(21) Anmeldenummer: 17803965.7
(22) Anmeldetag: 27.11.2017
(51) Int. Cl.: A61M 5/14

(54) **VORRICHTUNG ZUR ABGABE EINER SUBSTANZ**
DEVICE FOR DISPENSING A SUBSTANCE
DISPOSITIF DE DISTRIBUTION D'UNE SUBSTANCE

(30) Priorität: 28.11.2016 EP 16200929
(43) Veröffentlichungstag der Anmeldung: 02.10.2019
(73) Patentinhaber: SHL Medical AG, 6302 Zug (CH)
(72) Erfinder: WEIBEL, Ludwig Daniel, 9104 Waldstatt (CH); EGLOFF, Christoph, 8224 Löhningen (CH)
(86) Internationale Anmeldenummer: PCT/EP2017/080535
(87) Internationale Veröffentlichungsnummer: WO 2018/096149

(56) Entgegenhaltungen:
- EP-A1- 3 003 426
- WO-A1-2009/098306
- WO-A1-86/06967
- WO-A2-02/24259
- WO-A2-2006/031500
- US-A- 3 451 393
- US-A1- 2001 025 168
- US-A1- 2009 093 792

## Beschreibung

Die vorliegende Erfindung betrifft eine Vorrichtung zur Abgabe einer Substanz, insbesondere an einen Patienten, gemäss dem Oberbegriff von Anspruch 1.

Bei der Verabreichung flüssiger Formulierungen pharmazeutischer Wirkstoffe ist es in den meisten Fällen erforderlich, wohl definierte Substanzmengen abzugeben. Häufig müssen Arzneimittel dabei in den Körper eines Patienten injiziert werden. Zur parenteralen Injektion kommen üblicherweise Injektionsspritzen, Arzneimittelstifte (sogenannte Pens) oder Arzneimittelpumpen zum Einsatz.

Insbesondere Präparate, die in flüssiger Darreichungsform nicht über einen längeren Zeitraum gelagert werden können, stellen erhöhte Anforderungen an die Verabreichung. So sind insbesondere biotechnologisch hergestellte Wirkstoffe oft nur in gefriergetrockneter Form über einen längeren Zeitraum haltbar. Derartige Lyophilisate werden erst kurz vor deren Verabreichung in einer Injektionslösung, beispielsweise einer wässrigen Kochsalzlösung, gelöst und dann beispielsweise in eine Spritze aufgezogen. Allerdings kann ein derartiges Vorgehen auch bei Präparaten erforderlich sein, die ein Vermischen zweier Flüssigkeiten erfordern.

Im Stand der Technik sind bereits einige Vorrichtungen bekannt, um derartige Injektionslösungen aufzubereiten. So beschreibt die WO 2016/034683 Al einen Pen, mit dem ein Patient die Rekonstitution und Verabreichung eines Lyophilisates selbst durchführen kann. Der besagte Pen ist zum Einsatz mit einer sog. Doppelkammerkarpule ausgelegt. Eine solche gleicht im Wesentlichen einer gewöhnlichen Standardkarpule, weist allerdings zwei verschiebbar im Karpulenkörper gelagerte Stopfen auf, wodurch innerhalb desselben zwei hintereinander angeordnete Kompartimente gebildet werden. In diesen werden der lyophilisierte pharmazeutische Wirkstoff bzw. die Injektionslösung bereitgestellt. Durch Betätigung des Pens können die beiden Kompartimente miteinander in Fluidverbindung gebracht werden, wodurch es zu einem überströmen der Injektionslösung in das Kompartiment mit dem Lyophilisat, und damit zu einem Auflösen desselben kommt. Durch weitere Betätigung des Pens kann die Abgabe des rekonstituierten Wirkstoffes über eine Injektionskanüle erzielt werden. Ein derartiger Pen erleichtert die Handhabung des Wirkstoffes gegenüber einer herkömmlichen Injektionsspritze erheblich.

Ein Nachteil eines derartigen Systems besteht allerdings darin, dass aufgrund der sequentiellen Anordnung der beiden Kompartimente entlang der Längsachse der Doppelkammerkarpule der Pen vergleichsweise unhandlich ist. Dies stellt insbesondere ein Problem bei Indikationen dar, bei denen ein Patient ein Arzneimittel über einen längeren Zeitraum bei sich tragen muss, da er dieses bei Eintritt eines aufgrund seiner Disposition zu erwartenden medizinischen Notfalls benötigt. Bei einem derartigen Notfall kann es sich beispielsweise um einen anaphylaktischen Schock, einen Schlaganfall, einen epileptischen Anfall oder einen Herzinfarkt handeln. Ein weiterer Nachteil des oben beschriebenen Pens liegt darin, dass die eigentliche Injektion vom Patienten oder einer anwesenden Hilfsperson selbst vorgenommen werden muss. Gerade bei Eintritt eines medizinischen Notfalls ist für den Patienten die Ausübung einer Tätigkeit, in welcher er selbst höchstens laienhaft geschult ist, deutlich erschwert. Zusätzlich ist mit einem oben beschriebenen Pen keine, bzw. keine kontrollierte, Rekonstitution unabhängig von der Orientierung desselben relativ zur Schwerkraft möglich.

Ebenso im Stand der Technik findet sich WO 86/06967. Die Druckschrift beschäftigt sich mit einer automatischen Injektionsvorrichtung zur Rekonstitution eines Lyophilisates vor der subkutanen Abgabe. In der genannten Druckschrift wird ein Nasenteil des Geräts auf die Haut aufgebracht und dann eine Aktivierungstaste gedrückt. Zufolge Lösen einer komprimierten Feder wird Kraft auf ein Kolben- und Gehäusesystem ausgeübt, wodurch unter anderem die Injektionsnadel durch eine elastische Hülle hindurch aus dem Nasenteil austritt und ins Muskelgewebe des Patienten eindringt. Usability Studien haben aber gezeigt, dass dieser Schritt die Patienten erstens Überwindung kostete und dass zweitens der Verbleib einer steifen Kanüle, insbesondere bei langer Injektionszeit oder bei häufiger Penetration der Haut zur Verabreichung von Medizinalprodukten, für die Patienten unangenehm sein kann.

Es ist daher die Aufgabe der vorliegenden Erfindung, die Nachteile im Stand der Technik zu überwinden. Insbesondere ist es eine Aufgabe der vorliegenden Erfindung, eine Vorrichtung zur Abgabe einer Substanz, insbesondere an einen Patienten zu schaffen, deren Handhabung vereinfacht ist. Dies bezieht sich insbesondere auf den Transport der Vorrichtung, wenn diese auf dem Körper einer Person getragen werden muss. Allerdings soll je nach Ausführung auch die eigentliche Handhabbarkeit der Vorrichtung bei der Abgabe einer Substanz, insbesondere an einen Patienten, vereinfacht werden. Durch die Verwendung eines Systems aus Einstech- und Verweilkanüle soll die Injektion schmerzfreier, schonender und sicherer werden.

Diese Aufgaben werden durch eine Vorrichtung gelöst, welche die Merkmale in Anspruch 1 aufweist. Die Vorrichtung zur Abgabe einer Substanz, insbesondere an einen Patienten, umfasst einen die Substanz enthaltenden zylinderförmigen ersten Behälter und einen eine Flüssigkeit enthaltenden zweiten Behälter. Darüber hinaus umfasst die Vorrichtung eine Injektionsvorrichtung sowie Transfermittel zum Transferieren der Flüssigkeit vom zweiten zum ersten Behälter und vom ersten Behälter zur Injektionsvorrichtung. Der erste Behälter weist an einem ersten Ende ein Anschlusselement und einen zwischen dem ersten und einem zweiten Ende verschiebbaren Kolben auf. Durch Verschieben des Kolbens gegen das erste Ende des ersten Behälters ist eine darin enthaltene Flüssigkeit über die Injektionsvorrichtung abgebbar.

Die Injektionsvorrichtung kann zur Subkutanen, intramuskulären, intravenösen, intraarteriellen, intraartikulären, intrakardialen, intrakutanen, intraossären, intraperitonealen, intrapulmonalen, intrathekalen, intravitrealen oder intrazölomatischen Abgabe geeignet sein. Bei der Injektionsvorrichtung kann es sich um eine einfache Injektionskanüle oder wie im Folgenden noch weiter ausgeführt wird, auch um komplexere Vorrichtungen handeln.

Diese Ausgestaltung der Vorrichtung erlaubt vielfältige Möglichkeiten bei der Art des zweiten Behälters und bei der Positionierung desselben relativ zum Ersten. Dadurch kann eine weitaus kompaktere und bedarfsweise auch ergonomischere Vorrichtung bereitgestellt werden als die im Stand der Technik bekannten. Die Handhabbarkeit der Vorrichtung wird damit erhöht. Auch ist eine Rekonstitution eines Lyophilisates unabhängig von der Orientierung der Vorrichtung relativ zur Schwerkraft möglich.

Die Injektionsvorrichtung umfasst weiter eine Einstechkanüle. Zusätzlich umfasst die Injektionsvorrichtung eine Verweilkanüle, wobei in einer Ausgangsposition ein distaler Endbereich der Einstechkanüle koaxial im inneren der Verweilkanüle verläuft. Eine Injektionsvorrichtung mit Verweilkanüle ist dabei derart ausgelegt, dass sich die Einstechkanüle unmittelbar nach dem Einstechen wieder zurückzieht. Lediglich die Verweilkanüle verbleibt für einen kürzeren oder längeren Zeitraum in der Injektionsstelle. Dadurch kann einerseits die Unannehmlichkeit für einen Patienten reduziert werden. Andererseits besteht nach Gebrauch der Vorrichtung keine Verletzungsgefahr durch die Einstechkanüle. Ein zusätzlicher Nadelschutz entfällt.

Wenn die Injektionsvorrichtung nur eine Einstechkanüle umfasst, ist es allerdings auch denkbar, dass sich diese erst nach erfolgtem Injektionsvorgang selbständig wieder zurückzieht. Wenn die Vorrichtung am Körper eines Patienten angebracht wird, ist auch ein selbständiges zurückziehen der Einstechkanüle bei Entfernen der Vorrichtung vom Körper des Patienten denkbar.

Weiter hat die erfindungsgemässe Vorrichtung den Vorteil, dass nach dem Mischen der Karpulen-Inhalte durch eine Aktion des Anwenders und Anbringen der Vorrichtung am Körper nur noch eine zweite Operation des Anwenders zum Auslösen der Folgeschritte notwendig ist. Insbesondere kann die zweite Aktion im Drücken einer Taste bestehen. Durch Drücken der Taste kann das Aussetzen der Nadel, das Zurückziehen der Einstechkanüle und das Injizieren der Mischung in ein und derselben logischen Sequenz durchgeführt werden. Die rein mechanische Sequenz ist steuerbar durch das Verschieben eines Steuerungskamms gegenüber wenigstens einem Läufer und optional gegenüber weiteren Hebelelementen. Dadurch können in einer vordefinierten zeitlichen Abfolge mechanische Kräfte auf Bestandteile der Vorrichtung übertragen werden. Die vorliegende Erfindung kommt also mit einem Minimum an Handlungen seitens des Anwenders aus. Die Bedienung der Vorrichtung wird dadurch einfacher, angenehmer und weniger fehlergeneigt.

Die Injektionsvorrichtung kann einen ersten und einen zweiten verschiebbar gelagerten Läufer umfassen. Der erste Läufer kann mit der Einstechkanüle und der zweite Läufer mit der Verweilkanüle verbunden sein. Die Injektionsvorrichtung kann ferner ein über einen vordefinierten Steuerbereich bewegbares Steuerelement umfassen, das zum Verschieben des ersten Läufers und des zweiten Läufers mit diesen in Wirkverbindung bringbar ist. Das Steuerelement kann derart ausgebildet sein, dass es in einem ersten Teil des Steuerbereichs ein gleichgerichtetes, insbesondere simultanes, Verschieben der beiden Läufer, und damit ein Setzen der Verweilkanüle bewirkt. Das Steuerelement kann darüber hinaus derart ausgebildet sein, dass es in einem zweiten Teil des Steuerbereiches ein Blockieren des zweiten Läufers, und damit ein Halten der Verweilkanüle in einer Verweilposition, sowie ein Zurückverschieben des ersten Läufers, und damit ein Zurückziehen der Einstechkanüle aus dem distalen Endbereich der Verweilkanüle in eine Endposition, bewirkt.

Durch diese mechanische Ausführung der Injektionsvorrichtung kann ein durch die Einstechkanüle gestütztes Setzen der Verweilkanüle gefolgt von einem Zurückziehen der Einstechkanüle zuverlässig bewirkt werden. Der Mechanismus kommt mit einer geringen Anzahl von Einzelteilen aus, wodurch dieser kostengünstig herstellbar und zuverlässig in seiner Anwendung ist.

Die Läufer können über eine Führungsvorrichtung, vorzugsweise über eine gemeinsame Führungsvorrichtung, insbesondere über eine Linearführung verschiebbar gelagert sein. Der Einsatz einer gemeinsamen Führungsvorrichtung ermöglicht es, die Anzahl der Einzelteile der Injektionsvorrichtung weiter zu reduzieren. Insbesondere eine Linearführung ermöglicht eine Vereinfachung des Mechanismus.

So können die Läufer über eine gemeinsame Linearführung verschiebbar gelagert sein und die Linearführung in einer Setzrichtung parallel zu den distalen Endbereichen der Einstechkanüle und der Verweilkanüle ausgerichtet sein. Damit gibt die Linearführung nicht nur die Richtung des Verschiebens des ersten und des zweiten Läufers vor, sondern auch die Setzrichtung der Injektionsvorrichtung. Diese kann damit vorgängig definiert und der jeweiligen Anwendung der Vorrichtung angepasst werden. So ist es beispielsweise möglich, die Injektionsvorrichtung derart auszulegen, dass die Verweilkanüle im Wesentlichen senkrecht unter die Haut des Patienten gesetzt wird.

Das Steuerelement kann in einer Richtung senkrecht zu derjenigen der Linearführung bewegbar, insbesondere verschiebbar, gelagert sein. Dies ermöglicht eine optimale Kraftübertragung vom Steuerelement auf den ersten und den zweiten Läufer in beide Richtungen.

Das Steuerelement kann als verschiebbarer Kurventräger ausgebildet sein. Ein Kurventräger hat den Vorteil, dass mit ihm nahezu jede Bewegung, die über eine stetig-differenzierbare Funktion darstellbar ist, vorgegeben werden kann. Zusätzlich können durch ein passend gewähltes Steuerelement auch Nullstellen und Unstetigkeitsstellen einer Funktion dargestellt werden.

Das Steuerelement kann als beidseitig auf die Läufer wirkendes Paar von deckungsgleichen, verschiebbaren Kurventrägern ausgebildet sein. Dies ermöglicht es, die Kraft besonders wirkungsvoll vom Steuerelement auf die Läufer zu übertragen. Da die Kraftübertragung beidseitig erfolgt, kann verhindert werden, dass gleichzeitig ein Drehmoment auf die Läufer übertragen wird. Damit kann ein Verkanten derselben in einer Führungsvorrichtung vermieden werden.

Das Steuerelement kann einen ersten und einen zweiten Abschnitt umfassen. Der erste Abschnitt kann ein gleichgerichtetes, insbesondere simultanes, Verschieben der beiden Läufer, und damit ein Setzen der Verweilkanüle, bewirken. Der zweite Abschnitt kann ein Blockieren des zweiten Läufers, und damit ein Halten der Verweilkanüle in einer Verweilposition, sowie ein Zurückziehen des ersten Läufers, und damit ein Zurückziehen der Einstechkanüle aus dem distalen Endbereich der Verweilkanüle in eine Endposition, bewirken. Dabei können insbesondere unterschiedliche Seiten des zweiten Abschnittes auf den ersten und auf den zweiten Läufer wirken. Durch die Unterteilung des Steuerelementes in zwei Abschnitte kann dieses in Bezug auf seine Geometrie besonders einfach ausgestaltet werden.

Das Steuerelement kann über ein Federelement vorgespannt sein. Dies ermöglicht es, auf konstruktiv einfache Weise ein selbstständiges Verschieben des Steuerelementes zu bewirken, ohne dass dazu noch ein gesonderter Antrieb erforderlich wäre. Alternativ könnte das Steuerelement auch über expandierendes Gas angetrieben werden.

Das Steuerelement kann noch eine zusätzliche Kurvenfläche aufweisen, insbesondere in einem zweiten, dem Antrieb abgewandten Teil des Steuerbereichs. Diese Kurvenfläche kann bei weiter fortschreitender Bewegung mit einem in Bewegungsrichtung befindlichen Hebelelement in Wirkverbindung treten. Damit ist das Hebelelement von einer ersten Position in eine zweite Position bewegbar, wodurch eine weitere Funktion einer entsprechenden Vorrichtung ausgelöst werden kann. Beispielsweise kann eine Ventil- oder Fördervorrichtung, um ein Fluid zur Injektionsvorrichtung zu leiten, aktiviert werden.

Die Substanz in dem ersten Behälter kann ein Feststoff, insbesondere ein Lyophilisat, oder eine Flüssigkeit, insbesondere eine Lösung sein. Damit ist mit der beschriebenen Vorrichtung wahlweise ein Lösen eines Feststoffes, insbesondere die Rekonstitution eines Lyophilisates, oder auch ein Mischen zweier Flüssigkeiten möglich. Die Vorrichtung ist damit vielseitig einsetzbar.

Beim ersten Behälter kann es sich um eine Standardkarpule, ein Vial oder auch um eine herkömmliche Injektionsspritze handeln. Derartige Behälter sind im Stand der Technik etabliert und er füllen insbesondere im Falle von Pharmazeutika, welche üblicherweise einem Zulassungs- und Registrierungsverfahren unterliegen, die regulatorischen Anforderungen.

Der zweite Behälter kann ebenfalls zylinderförmig mit einem Anschlusselement an einem ersten Ende sowie einem zwischen dem ersten und dem zweiten Ende verschiebbaren Kolben ausgestaltet sein. Behälter dieser Art sind auch für Flüssigkeiten, beispielsweise in Form von Karpulen mit einer Injektionslösung, weit verbreitet und von einer Vielzahl von Anbietern kommerziell erhältlich. Damit kann bei der Verwendung einer derartigen Vorrichtung auf Standardkomponenten zurückgegriffen werden.

Allerdings ist es auch denkbar, den zweiten Behälter kollabierbar, beispielsweise in Form einer Tube, eines Beutels oder eines Faltenbalges, auszugestalten. Dies ermöglicht weitere Ausführungen einer erfindungsgemässen Vorrichtung, die insbesondere im Hinblick auf eine kompakte Bauweise vorteilhaft sind.

Bei zylinderförmiger Ausführung kann der zweite Behälter parallel, insbesondere gleichgerichtet zum Ersten angeordnet sein. Damit können mit der Vorrichtung bspw. zwei Karpulen verwendet werden. Durch ihre relative Anordnung fällt die Vorrichtung gesamthaft deutlich weniger sperrig aus als die im Stand der Technik bekannten.

Es versteht sich allerdings von selbst, dass die vorliegende Erfindung nicht auf zwei Behälter limitiert ist. So kann eine erfindungsgemässe Vorrichtung beispielsweise neben einem ersten Behälter mit einem Lyophilisat und einem zweiten Behälter mit einem Lösungsmittel auch einen dritten Behälter mit einem Verdünnungsmittel umfassen. Beim Lösungsmittel und beim Verdünnungsmittel handelt es sich dabei zumeist um wässrige Lösungen. Für die Ausgestaltung des dritten und jedes weiteren Behälters stehen die oben für den zweiten Behälter diskutierten Varianten zur Verfügung. Im Rahmen einer solchen Ausgestaltung ist es einerseits möglich, das Verdünnungsmittel ähnlich wie das Lösungsmittel vom dritten Behälter in den ersten Behälter, und dann weiter zur Injektionsvorrichtung, zu transferieren. Allerdings kann es auch vorteilhaft sein, wenn die Vorrichtung nicht, wie oben beschrieben, zum Transferieren der Flüssigkeit vom ersten Behälter zur Injektionsvorrichtung ausgelegt ist, sondern dass die Flüssigkeit erst vom ersten Behälter in den dritten Behälter, und von dort weiter zur Injektionsvorrichtung, transferiert wird.

Der Kolben des ersten Behälters, und gegebenenfalls auch der Kolben des zweiten Behälters, können durch Verschiebungsmittel verschiebbar sein. Damit kann ein selbstständiges Transferieren der Flüssigkeit vom zweiten zum ersten Behälter oder auch vom ersten Behälter zur Injektionsvorrichtung erzielt werden.

Der Kolben des ersten Behälters, und gegebenenfalls auch der Kolben des zweiten Behälters, können durch jeweils ein Federelement vorgespannt und damit verschiebbar sein. Federelemente stellen eine kostengünstige und zuverlässige Ausführung von Verschiebungsmitteln dar. Dies ist insbesondere von Bedeutung, wenn die Vorrichtung lediglich zum einmaligen Gebrauch vorgesehen ist.

Der Kolben des ersten Behälters, und gegebenenfalls auch der Kolben des zweiten Behälters, können über Rückhaltemittel gegen die Vorspannung zurückgehalten sein. Dabei versteht es sich von selbst, dass dies direkt oder indirekt realisiert werden kann; Das heisst die Rückhaltemittel müssen nicht zwingend auf einem Kolben, sondern können auch auf ein Federelement oder eine andere Komponente der Vorrichtung wirken. Durch den Einsatz von Rückhaltemitteln kann gewährleistet werden, dass ein Verschieben der Kolben durch die Vorspannung erst eintritt, wenn ein solches erwünscht ist. Gerade bei indirektem Wirken der Rückhaltemittel können das oder die Federelemente allerdings auch vorgespannt sein, bevor der erste Behälter, und gegebenenfalls auch der zweite Behälter, in die Vorrichtung eingesetzt wird/werden.

Allerdings kann der Kolben des ersten Behälters, und gegebenenfalls auch der Kolben des zweiten Behälters, über einen Linearantrieb, insbesondere einen Spindeltrieb oder ein Kurvengetriebe, verschiebbar sein. Dadurch kann eine elektronische Steuerung der Kolbenbewegung realisiert werden. Dies ist insbesondere vorteilhaft, da damit eine überaus genaue Dosierung der Substanz im Zuge ihrer Abgabe, bedarfsweise auch über einen längeren Zeitraum, erzielt werden kann.

Allerdings ist auch ein pneumatischer oder ein hydraulischer Antrieb einsetzbar. Ein pneumatischer Antrieb erlaubt dabei vielfältige Möglichkeiten in Bezug auf die Bereitstellung eines Pneumatikmediums. So kann dieses beispielsweise in einem Pneumatikdruckbehälter bereitgestellt werden. Allerdings ist auch der Aufbau eines Gasdrucks durch eine entsprechende chemische Reaktion (Gasexpansion) denkbar.

Das Anschlusselement des ersten Behälters, und gegebenenfalls auch dasjenige des zweiten Behälters, kann als Septum ausgebildet sein. Ein Septum ermöglicht ein besonders zuverlässiges und dichtes Abschliessen eines Behälters. Trotzdem kann der Behälter einfach angeschlossen werden.

Das Septum des ersten Behälters, und gegebenenfalls auch dasjenige des zweiten Behälters, kann/können mit Penetrationsmitteln, insbesondere durch Verschieben oder durch Torsion des ersten Behälters und gegebenenfalls auch des zweiten Behälters, gegen das/die Penetrationsmittel, penetrierbar sein. Es sind auch Ausführungsformen möglich, bei denen das Penetrationsmittel gegenüber dem Behälter verschiebbar ist und die Penetration durch diese Bewegung ausgeführt wird. Durch Penetrieren eines Septums kann ein Anschliessen des ersten Behälters, und gegebenenfalls auch des zweiten Behälters, an die Vorrichtung auf besonders zuverlässige und kostengünstige Weise erzielt werden.

Das oder die Penetrationsmittel können als, insbesondere schräg angeschliffene, Hohlkanüle/n ausgebildet sein. Damit ist ein direkter Transfer einer Flüssigkeit von einem Behälter in eine Hohlkanüle erzielbar. Weitere fluidführende Teile zum Anschluss des Behälters entfallen.

Um das Risiko einer Kontamination der abzugebenden Substanz zu verringern, ist es insbesondere bei Sterilanwendungen Vorteilhaft, wenn die Penetrationsmittel jeweils mit einer flexiblen Schutzkappe oder Schutzhülse versehen sind, die ebenfalls zusammen mit einem Septum penetriert wird. Der Einsatz einer derartigen Schutzkappe oder Schutzhülse erlaubt insbesondere eine Endmontage der Vorrichtung unter nicht sterilen Bedingungen.

Das Septum des ersten Behälters, und gegebenenfalls auch dasjenige des zweiten Behälters, kann durch Verschieben, insbesondere mit den Verschiebungsmitteln für den oder die Kolben des ersten und/oder zweiten Behälters penetrierbar sein. Bei dieser Ausführung wird eine Bewegung von fluidführenden Teilen innerhalb der Vorrichtung vermieden. Darüber hinaus können die Verschiebungsmittel gleichzeitig zum Verschieben des oder der Kolben und zum Anschliessen des oder der Behälter an die Vorrichtung genutzt werden.

Der erste Behälter kann über das Anschlusselement mit dem zweiten Behälter und der Injektionsvorrichtung in Fluidkommunikation bringbar sein. Dadurch, dass jeglicher Fluidtransfer vom oder zum ersten Behälter über das Anschlusselement realisiert wird, entfällt das Anbringen von weiteren Fluidverbindungen.

Der erste Behälter ist über eine Ventilanordnung wahlweise mit dem zweiten Behälter oder der Injektionsvorrichtung in Fluidkommunikation bringbar. Dadurch lassen sich die Fluidströme vom oder zum ersten Behälter gezielt steuern.

Die Injektionsvorrichtung, und gegebenenfalls auch die Verschiebungsmittel, können über Aktivierungsmittel aktivierbar sein, wobei die Aktivierungsmittel insbesondere zum Lösen der Rückhaltemittel ausgelegt sind. Durch den Einsatz von Aktivierungsmitteln kann die Betätigung der Vorrichtung, insbesondere für einen Patienten, erleichtert werden.

Die Injektionsvorrichtung und die Verschiebungsmittel zum Verschieben des ersten Kolbens können über dasselbe Aktivierungsmittel aktivierbar sein. Damit kann durch Betätigung eines einzelnen Aktivierungsmittels der gesamte Injektionsvorgang aktiviert werden.

Es versteht sich von selbst, dass auch das Steuerelement über Rückhaltemittel gegen die Vorspannung zurückgehalten sein kann. Wie bereits erläutert, können diese Rückhaltemittel über Aktivierungsmittel, insbesondere über dieselben Aktivierungsmittel wie für die oben beschriebenen Rückhaltemittel, lösbar sein.

Allerdings kann das Steuerelement auch über ein Schraubengetriebe, insbesondere über einen Spindeltrieb, bewegbar sein. Dadurch kann das Steuerelement leicht durch einen Drehantrieb mit der erforderlichen Untersetzung bewegt werden.

Das Steuerelement kann noch weitere Kurvenflächen zum Auslösen weiterer Mechanismen aufweisen. Beispielsweise kann das Steuerelement in einem zweiten Teil des Steuerbereiches auf ein Hebelelement einwirken, wodurch das Hebelelement verschoben oder rotiert wird. Damit kann eine weitere Funktion einer entsprechenden Vorrichtung kontrolliert werden, beispielsweise die Aktivierung einer Ventil- oder Fördervorrichtung, um ein Fluid zur Injektionsvorrichtung zu leiten.

Die Vorrichtung kann auch noch weitere Kurvenflächen aufweisen, die nicht direkt am Steuerelement angebracht sind. Beispielsweise kann eine Kurvenfläche einstückig mit einem Anschlagelement ausgebildet sein, welches über eine Stange mit dem Steuerelement verbunden ist. Beim Verschieben des Steuerelements kann eine solche Kurvenfläche dem Steuerelement folgen und im Zuge der Bewegung in einen Schieber eingreifen. Dadurch kann etwa ein weiteres Rückhaltemittel gelöst werden und ein zuvor in gespannter Position festgehaltenes Verschiebemittel kann sich entspannen.

In einer Ausführungsform kann der zweite Läufer als Halteblech ausgeführt sein. Ein Halteblech hat den Vorteil, dass es im Vergleich zu einem Spritzgussteil einfacher fertigbar ist. Zudem weist es bei gleicher Wandstärke eine grössere mechanische Belastbarkeit auf. Ausserdem kann es, falls erwünscht, eine gewisse Federwirkung zeigen.

Ein Übergang von der Einstechkanüle zur Verweilkanüle kann mit einem Dichtungselement abgedichtet sein, welches in der Verweilposition gequetscht wird, insbesondere durch einen Wandabschnitt eines Dosiergerätes und den zweiten Läufer. Damit kann ein besonders fluiddichter Übergang von der Einstechkanüle zur Verweilkanüle erzielt werden. Es versteht sich von selbst, dass diese Ausführung der Injektionsvorrichtung auch unabhängig von anderen Merkmalen der Vorrichtung realisiert werden kann.

Die Einstechkanüle kann einstückig als durchgehende Hohlnadel, insbesondere aus Stahl, ausgebildet sein. Diese Ausführung ermöglicht es, dass die Einstechkanüle gleichzeitig eine Leitungsfunktion und eine Penetrationsfunktion wahrnimmt. Insbesondere Dichtungsprobleme zwischen verschiedenen Leitungsabschnitten können damit vermieden werden. Der proximale Endbereich der Einstechkanüle kann ortsfest und starr angeordnet sein. Damit kann eine zuverlässige Fluidverbindung zum Beispiel an die Ventilvorrichtung gewährleistet werden.

Die Vorrichtung kann ein Gehäuse mit einer aussenseitigen Kontaktfläche, über welche die Vorrichtung insbesondere an den Körper eines Patienten anbringbar ist, umfassen. In einer bevorzugten Ausführungsform ist die Vorrichtung über ein selbstklebendes Pflaster an den Körper anbringbar. Das selbstklebende Pflasterkann bereits an der aussenseitigen Kontaktfläche der Vorrichtung angebracht sein und durch Abziehen einer Schutzfolie freigelegt werden. Durch eine derartige Ausgestaltung der Vorrichtung kann eine Injektion auf besonders zuverlässige und anwenderfreundliche Weise gewährleistet werden. Zudem ist dadurch die Abgabeeiner Substanz nach einem vordefinierten Schema und/oder über einen längeren Zeitraum, insbesondere bei einem mobilen Patienten, möglich. Je nach Indikation ist aber auch die Abgabe einer Substanz an einen Patienten denkbar, wenn dieser, beispielsweise aufgrund eines akuten medizinischen Notfalls, nicht mehr zu einer selbstständigen Injektion in der Lage ist.

Die Einstechkanüle, und gegebenenfalls auch die Verweilkanüle, kann/können in der Ausgangsposition im Wesentlichen innerhalb des Gehäuses angeordnet und durch eine Setzöffnung in der Kontaktfläche aus dem Gehäuse ausfahrbar sein. Dadurch kann insbesondere bei einer an dem Körper eines Patienten angebrachten Vorrichtung eine Injektion vollautomatisch erfolgen. Dies erleichtert die Handhabung der Vorrichtung durch einen Patienten.

Ein weiterer Aspekt der vorliegenden Offenbarung betrifft die Verwendung einer oben beschriebenen Vorrichtung zur Abgabe einer Substanz, insbesondere an einen Patienten.

Beschrieben wird nachstehend auch ein Verfahren zum Befüllen eines Behälters mit einem Lyophilisat. Der Behälter ist zylinderförmig ausgestaltet und umfasst einen zwischen einem ersten und einem zweiten Ende verschiebbaren Kolben. Insbesondere kann es sich beim besagten Behälter um eine Karpule handeln. Der Kolben ist zu Beginn des Verfahrens derart zwischen dem ersten und dem zweiten Ende positioniert, dass sich zwischen dem Kolben und dem ersten Ende ein Volumen zur Aufnahme einer Flüssigkeit ergibt. Das Volumen wird zumindest teilweise mit einer Lösung, insbesondere einer wässrigen Lösung, enthaltend eine Substanz gefüllt. Darauf wird der Behälter gekühlt, derart dass die Lösung vom flüssigen in den festen Aggregatszustand übergeht. Durch Anlegen eines Vakuums auf beiden Seiten des Kolbens kommt es zu einer Sublimation des Lösungsmittels, welches am ersten Ende aus dem Behälter entweicht. Die nicht flüchtigen Anteile der Lösung verbleiben als Lyophilisat im Behälter. Nach Abschluss des Sublimationsvorgangs wird der Behälter an seinem ersten Ende mit einem Anschlusselement, insbesondere mit einem Septum verschlossen.

Bei einer ersten Variante des Verfahrens wird der Behälter nach dem Verschliessen wieder unter Atmosphärendruck gesetzt, wobei der Kolben im Behälter frei verschiebbar ist. Durch den entstehenden Druckunterschied auf den beiden Seiten des Kolbens wird dieser gegen das erste Ende des Behälters verschoben.

Bei einer zweiten Variante des Verfahrens wird der Behälter nach dem Verschliessen wieder unter Atmosphärendruck gesetzt, wobei der Kolben nicht frei im Behälter verschiebbar, beispielsweise über eine Kolbenstange gehalten, ist. Dadurch verbleibt im Kolben ein Vakuum, bzw. ein Unterdruck.

Das Wiederaufwärmen des Behälters kann vor oder nach dem Wechsel auf Atmosphärendruck erfolgen.

Das beschriebene Verfahren hat den Vorteil, dass auf zuverlässige und effiziente Weise ein Behälter, insbesondere eine Karpule, gefüllt mit einem Lyophilisat bereitgestellt werden kann, der im Wesentlichen frei von einem Gas, insbesondere frei von Luft ist. Ein solcher Behälter ist insbesondere in Verbindung mit einer oben beschriebenen Vorrichtung zur gasfreien bzw. luftfreien Rekonstitution des Lyophilisates vorteilhaft.

Das erfindungsgemässe Steuerelement der Vorrichtung kann ausserdem als verschiebbarer Kurventräger ausgebildet sein. Das Steuerelement kann insbesondere einen linear verschiebbaren Schlitten umfassen, an dem wenigstens ein Kurventräger ausgebildet ist. Besonders bevorzugt verläuft die Verschieberichtung des Schlittens parallel zu den Längsachsen des ersten und zweiten Behälters.

Der Einsatz eines verschiebbaren Kurventrägers und insbesondere eines linear verschiebbaren Schlittens mit einem oder mehreren Kurventrägern hat den Vorteil, dass die zeitlich korrekte Abfolge der Schritte auf einfache und fehlerresistente Weise gewährleistet wird.

Die Sequenz, welche durch den verschiebbaren Schlitten gesteuert wird, wird aufgrund der nachstehend beschriebenen Zeichnungen noch klarer. zusammenfassend kann die Sequenz des Steuerelements die folgenden Schritte umfassen: Erstens werden zwei Läufer, wovon einer mit der Einstechkanüle und der andere mit der Verweilkanüle in Wirkverbindung steht, gemeinsam Richtung Gewebe des Patienten geschoben. zweitens wird der Läufer, der mit der Verweilkanüle in Wirkverbindung steht in der Position arretiert, während der Läufer, der mit der Einstechkanüle in Wirkverbindung steht, durch eine Kurvenfläche wieder vom Gewebe des Patienten weg und weiter ins Innere des Gehäuses gelenkt wird. Drittens kann eine zusätzliche Kurvenfläche des Kurventrägers mit einem Hebelelement in Wirkverbindung treten, wodurch das Hebelelement verschoben wird und/oder es zu einer Rotation um die Drehachse des Hebelelements kommt. Das Hebelelement kann damit weitere Funktionen einer entsprechenden Vorrichtung kontrollieren, etwa eine Aktivierung einer Ventil- oder Fördervorrichtung. Viertens kann gleichzeitig oder kurz nach der Betätigung des Hebelelements die Injektion ausgelöst werden, etwa durch das Lösen einer Feder, welche zuvor durch ein verschiebbares Rückhalteelement in gespannter Position festgehalten wurde. Die Sequenz wird durch die Wirkfolge des Steuerelements getaktet und automatisiert. Zwei konkrete Ausführungsbeispiele gehen aus den nachstehend beschriebenen Zeichnungen hervor. Die Verschieberichtung entlang den Längsmittelachsen kann gewählt werden, um eine platzsparende und damit für den Anwender handliche und einfach zu transportierende Vorrichtung anzubieten.

Weitere Vorteile und einzelne Merkmale der Erfindung ergeben sich aus der nachfolgenden Beschreibung zweier Ausführungsbeispiele und aus den Zeichnungen.

Es zeigen schematisch:
- Figuren 1 bis 6:: Perspektivische Darstellungen eines ersten Ausführungsbeispiels einer erfindungsgemässen Vorrichtung in verschiedenen Betriebszuständen;
- Figuren 7 und 8:: Perspektivische Darstellungen einer Variante des Ausführungsbeispiels gemäss den Figuren 1 bis 6 in verschiedenen Betriebszuständen;
- Figuren 9 bis 11:: Perspektivische Schnittansichten einer bevorzugten Ausführungsform der Vorrichtung gemäss den Figuren 1 bis 8 zur Verdeutlichung des Arbeitsprinzips der Injektionsvorrichtung;
- Figur 12:: Perspektivische Darstellung eines alternativen Ausführungsbeispiels einer erfindungsgemässen Vorrichtung;
- Figuren 13 bis 17:: Perspektivische Darstellung eines alternativen Ausführungsbeispiels einer erfindungsgemässen Vorrichtung in verschiedenen Betriebszuständen;

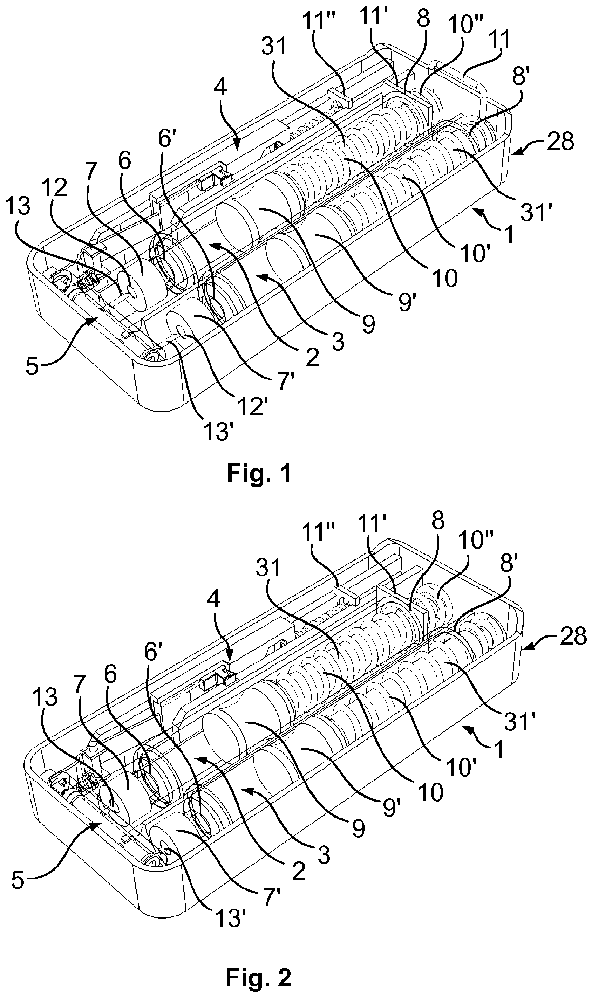

Wie aus Figur 1 hervorgeht, weist ein erstes Ausführungsbeispiel einer erfindungsgemässen Vorrichtung 1 einen ersten Behälter 2 und einen zweiten Behälter 3, jeweils in Form einer Karpule, auf. Zur besseren Verständlichkeit sind bei den Behältern 2, 3 die Karpulenkörper transparent dargestellt. Darüber hinaus umfasst die Vorrichtung 1 eine Injektionsvorrichtung 4 und Transfermittel 5. Auch das Aussengehäuse der Transfermittel 5 ist zum besseren Verständnis transparent dargestellt. Die beiden Behälter 2 und 3 weisen jeweils ein erstes Ende 6, 6' und ein zweites Ende 8, 8' auf. Innerhalb der Behälter 2 und 3 ist jeweils ein Kolben 9, 9' zwischen den Enden 6, 6' und 8, 8' verschiebbar gelagert. An den Enden 6 und 6' sind die Behälter 2 und 3 jeweils mit einem Anschlusselement 7, 7' abgeschlossen. Die Kolben 9, 9' stehen indirekt über die Kolbenstangen 31, 31' unter Vorspannung und sind durch die Federelemente 10, 10' verschiebbar. Die Kolben 9, 9' sind durch die Rückhalteelemente 11, 11' zurückgehalten, welche jeweils an den Kolbenstangen 31, 31' angreifen. Am ersten Behälter 2 greifen dabei zwei Federelemente 10 und 10'' an. Während das Federelement 10 zum Verschieben des Kolbens 9 dient, hat das Federelement 10'' lediglich die Funktion ein Ver schieben des ersten Behälters 2 zu bewirken. Die Federelemente 10 und 10'' werden selektiv über die Rückhaltemittel 11 und 11' zurückgehalten. Die Anschlusselemente 7, 7' des ersten und des zweiten Behälters 2, 3 weisen jeweils ein Septum 12, 12' auf. Die Septen 12, 12' sind durch die Penetrationselemente 13, 13' penetrierbar. Die Vorrichtung 1 ist in einem Gehäuse 28 untergebracht.

Die Figur 2 zeigt die Vorrichtung 1 gemäss Figur 1 nachdem das Rückhaltemittel 11 entfernt wurde. Es ist zu erkennen, dass es durch die Federelemente 10', 10' zu einem Verschieben der Behälter 2, 3, und damit zu einem Penetrieren der Septen 12, 12' durch die Penetrationselemente 13, 13' kam. Die Kolben 9, 9' innerhalb der Behälter 2, 3 sind zu diesem Zeitpunkt noch unverschoben.

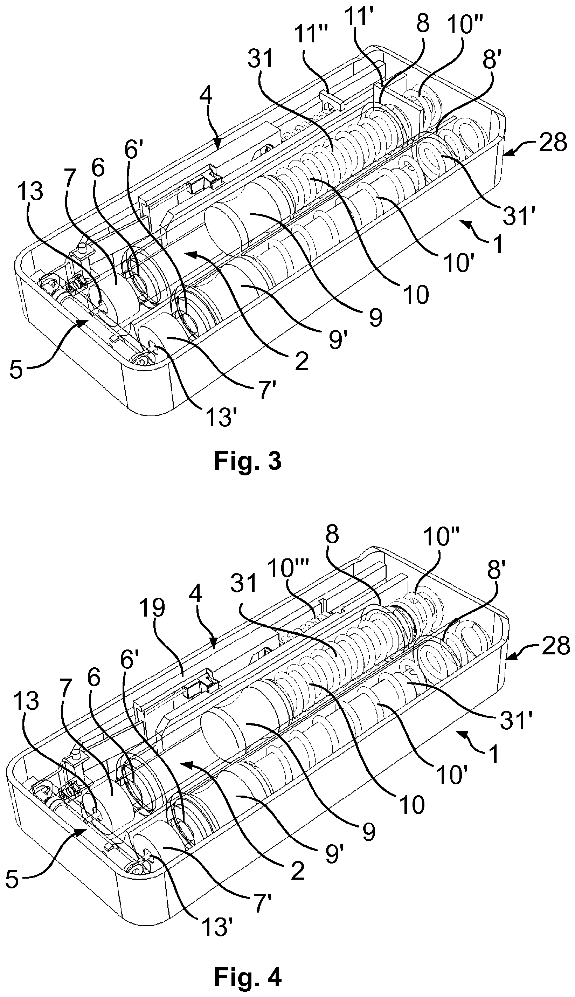

Die Figur 3 zeigt die Vorrichtung 1 gemäss den Figuren 1 und 2 nachdem der Kolben 9' des zweiten Behälters 3 durch das Federelement 10' gänzlich zum ersten Ende 6' des zweiten Behälters 3 verschoben wurde. Dadurch kam es zu einem Transfer der zuvor im Behälter 3 vorhandenen Flüssigkeit (nicht gezeigt) über die Transfermittel 5 in den ersten Behälter 2. Der erste Behälter 2 stand zuvor unter Vakuum und enthielt ein Lyophilisat (nicht gezeigt). Entsprechend konnte der erste Behälter 2 den gesamten Inhalt des zweiten Behälters 3 aufnehmen, ohne dass ein Verschieben des Kolbens 9 erforderlich war. Dadurch kommt es zu einem Auflösen des Lyophilisates in der Flüssigkeit. Um ein unbeabsichtigtes Verschieben des Kolbens 9 des ersten Behälters zu verhindern, wenn dieser unter Vakuum steht, ist der Kolben 9 fest mit der Kolbenstange 31 verbunden.

In Figur 4 sind die Rückhaltemittel 11', 11'' zusätzlich entfernt. Das Rückhaltemittel 11' hat zuvor den Kolben 9 des ersten Behälters 2 zurückgehalten, der über das Federelement 10 vorgespannt war. Das Rückhaltemittel 11'' hat zuvor das Federelement 10''', über welches die Injektionsvorrichtung 4 vorgespannt war, zurückgehalten.

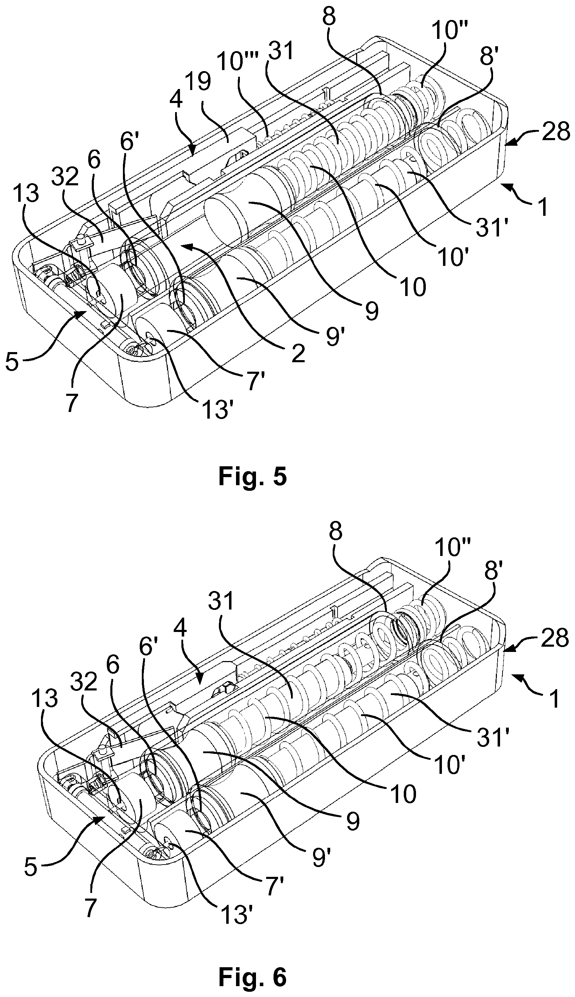

Die Figur 5 zeigt die Vorrichtung 1 nach teilweisem Verschieben des Steuerelementes 19 der Injektionsvorrichtung 4 durch das Federelement 10‴. Das Steuerelement 19 wirkt dadurch auf den Hebel 32, wodurch es zu einem Umschalten der Ventilvorrichtung der Transfermittel 5 kommt. Während der erste Behälter 2 in einer ersten Phase mit dem zweiten Behälter 3 in Fluidkommunikation steht, steht der erste Behälter 2 später mit der Injektionsvorrichtung 4 in Fluidkommunikation. Dadurch dass der Fluidweg vom ersten Behälter 2 zur Injektionsvorrichtung freigegeben wird, kommt es zu einem Druckabfall im ersten Behälter 2, wodurch sich nun auch der Kolben 9 des ersten Behälters 2 in Bewegung setzen kann.

In Figur 6 ist das Verschieben des Kolbens 9 des ersten Behälters 2 in seine Endposition abgeschlossen. Dadurch wurde die gesamte im ersten Behälter 2 vorhandene Flüssigkeit über die Injektionsvorrichtung 4 abgegeben.

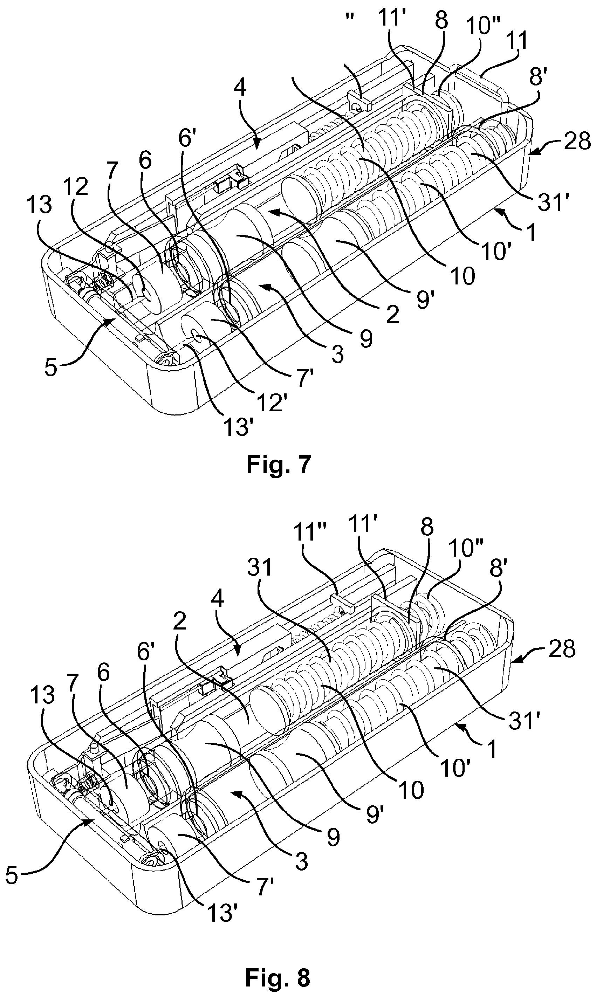

In den Figuren 7 und 8 ist eine Variante des ersten Ausführungsbeispiels der vorliegenden Erfindung gemäss den Figuren 1 bis 6 gezeigt. Die Figuren 7 und 8 entsprechen dabei den Figuren 1 und 2. In Bezug auf den Weiteren Betriebsablauf der Vorrichtung gemäss den Figuren 3 bis 6 sind die beiden Varianten des Ausführungsbeispiels identisch.

Wie in Bezug auf die oben diskutierte Variante erläutert wurde, ist der erste Behälter 2, in welchem das Lyophilisat (nicht gezeigt) bereitgestellt wird, anfangs unter Vakuum. Dies ist insbesondere von Bedeutung, wenn eine möglichst gasfreie, insbesondere Luftfreie, Rekonstitution erzielt werden soll. Eine alternative Lösung zu diesem Problem ist in Figur 7 gezeigt, wobei der Kolben 9 hier nahezu am ersten Ende 6 des ersten Behälters 2 anliegt und damit das im ersten Behälter 9 verbliebende Gasvolumen minimiert. Das verbleibende Volumen im ersten Behälter 2 ist derart gewählt, dass dieser gerade noch das Lyophilisat (nicht gezeigt) aufnehmen kann. Im Gegensatz zu der vorgängig besprochenen Variante ist der Kolben 9 des ersten Behälters nicht mit der Kolbenstange 31 verbunden, sondern frei im ersten Behälter 9 verschiebbar.

Die Figur 8 zeigt analog zur Figur 2 die Vorrichtung 1 nachdem das Rückhaltemittel 11 entfernt wurde. Es kommt in der Folge ebenfalls zu einem Transfer der im zweiten Behälter 3 enthaltenen Flüssigkeit über die Transfermittel 5 in den ersten Behälter 2. Der Endzustand dieses Transfers ist in Figur 3 gezeigt. Es ist zu erkennen, dass der Kolben 9 des ersten Behälters gegen die Kolbenstange 31 verschoben wurde und nun an dieser anliegt.

Ein Vorteil dieser Variante liegt darin, dass der erste Behälter 2 nicht mit Vakuum bzw. Unterdruck bereitgestellt werden muss. Damit wird das Risiko einer Kontamination dessen Inhaltes, durch Ansaugen von Umgebungsluft erheblich reduziert.

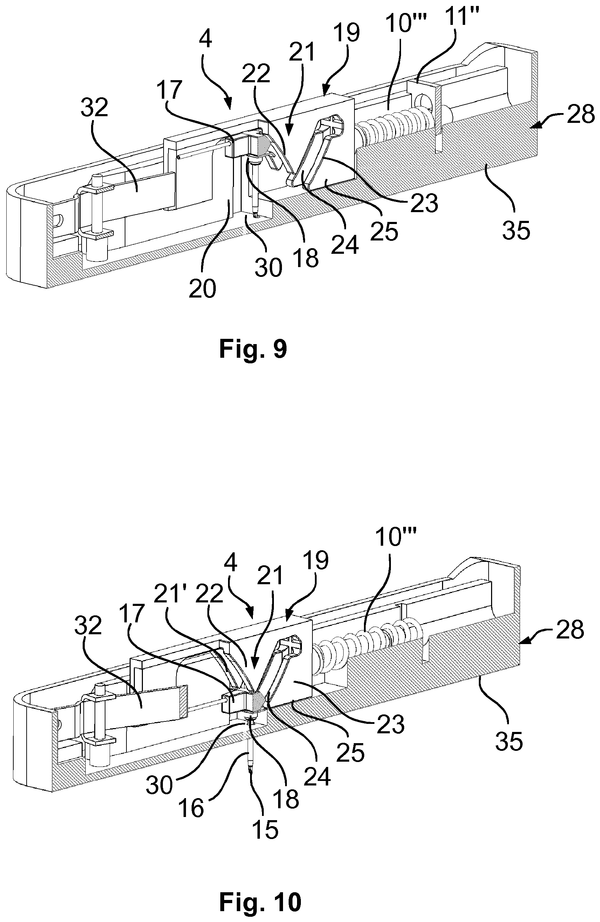

In Figur 9 ist die Injektionsvorrichtung 4 besser ersichtlich. Diese umfasst das Steuerelement 19, welches mit einem ersten Läufer 17 und einem zweiten Läufer 18 in Wirkverbindung bringbar ist. Das Steuerelement 19 ist über das Federelement 10‴ vorgespannt. Es wird allerdings durch ein Rückhaltemittel 11'' entgegen der Vorspannung zurückgehalten. Ferner ist eine Linearführung 20 ersichtlich, mit welcher der erste Läufer 17 und der zweite Läufer 18 geführt sind. Das Steuerelement 19 ist als Paar von deckungsgleichen, verschiebbaren Kurventrägern 21, 21' ausgebildet (Figur 10). Vom vorderen Kurventräger 21 sind beide Abschnitte 22 und 23 sichtbar. Es ist zu erkennen, dass eine Einstechkanüle 15 in der Ausgangsposition der Injektionsvorrichtung 4 innerhalb einer Verweilkanüle 16 verläuft (Figur 10). An der Unterseite des Gehäuses 28 ist eine Setzöffnung 30 angebracht, welche eine Kontaktfläche 29 des Gehäuses 28 durchdringt. Der zweite Läufer 18 ist als Halteblech ausgeführt, an welchem eine Dichtung angebracht ist, die den Übergang zwischen der Setzkanüle 15 und der Verweilkanüle 16 abdichtet.

In Figur 10 sind der erste Läufer 17 und der zweite Läufer 18 an ihrem unteren Anschlagspunkt angekommen. Die Einstechkanüle 15 ist damit gesetzt. Es ist hier deutlich zu erkennen, dass sich das Steuerelement 19 aus zwei Kurventrägern 21, 21' zusammensetzt. Das Steuerelement 19 hat hier erst etwa die Hälfte seines vorgesehenen Weges zurückgelegt. Es kann sich durch die Federvorspannung weiter verschieben. Von hier an wirkt jeweils eine erste Seite 24 des jeweils zweiten Abschnittes 23 der Kurventräger 21, 21' auf den ersten Läufer 17 und jeweils eine entsprechende Seite 25 auf den zweiten Läufer 18.

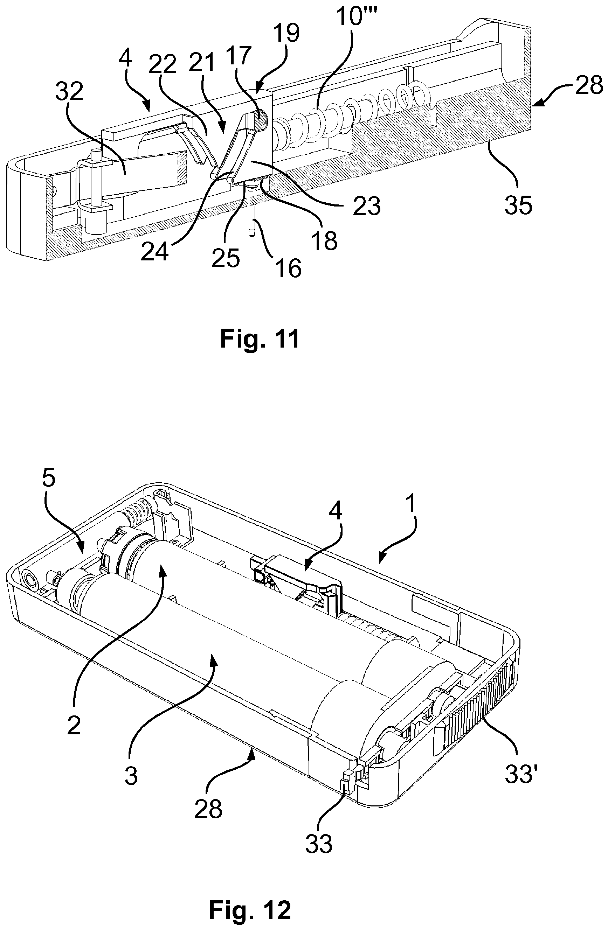

In Figur 11 hat das Steuerelement 19 seinen Anschlag erreicht. Es ist nicht mehr weiter durch das Federelement 10‴ bewegbar. Der erste Läufer 17 hat dadurch seine Endposition erreicht. Der zweite Läufer 18, und damit die Verweilkanüle 16, sind in der Verweilposition arretiert. Auch der Hebel 32 ist durch die Wirkung des Steuerelementes 19 in seiner Endposition angelangt, wodurch es zu einem Umschalten der Ventilvorrichtung der Transfermittel 5 kam.

Figur 12 zeigt ein alternatives Ausführungsbeispiel einer erfindungsgemässen Vorrichtung 1. Bei dieser Ausführung bietet das Gehäuse 28 im Bereich der Injektionsvorrichtung 4 Raum für Elektronik-Komponenten, welche hier jedoch nicht gezeigt sind. In dieser Ausführung lässt sich die Vorrichtung 1 durch die Aktivierungsmittel 33, 33' aktivieren, welche als einfach zu betätigende Hebel oder Schieber ausgebildet sind.

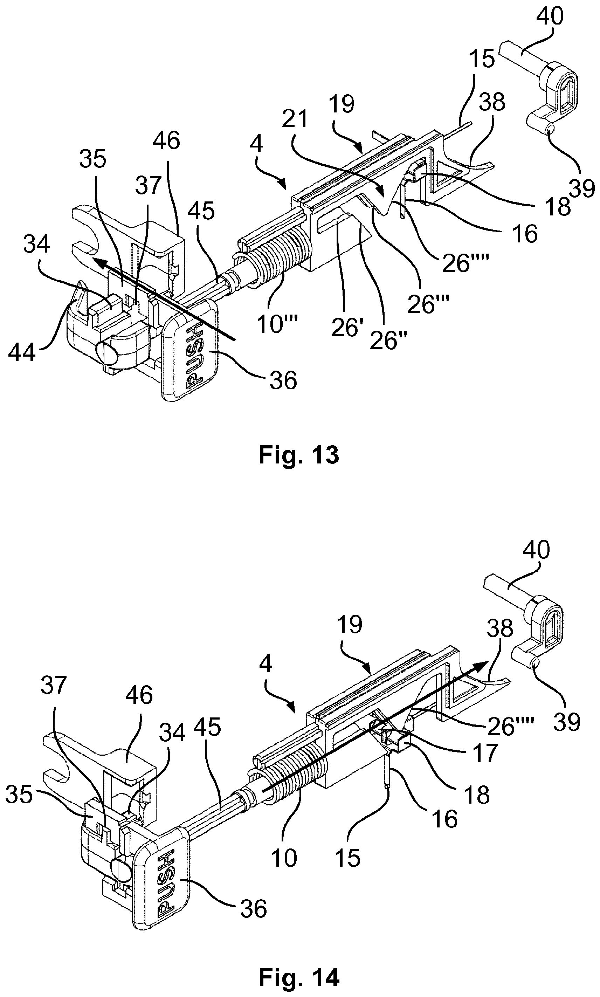

Die Figuren 13 bis 16 zeigen ein weiteres alternatives Ausführungsbeispiel einer erfindungsgemässen Injektionsvorrichtung 4.

In Figur 13 ist die besagte Vorrichtung 4 in ihrer Ausgangsposition ersichtlich. Das Steuerelement 19 ist über ein Federelement 10''', das hier als Schraubenfeder ausgebildet ist, vorgespannt. Das Steuerelement 19 wird durch ein Anschlagelement 34, welches auf einer Anschlagplatte 35 aufliegt, gegen die Vorspannung zurückgehalten. Durch Betätigen der Auslösetaste 36 (in Pfeilrichtung) wird die Anschlagplatte 35 in einer Richtung senkrecht zur Verschiebungsrichtung des Steuerelementes 19 verschoben. Dadurch kann das Anschlagelement 34 durch die Aussparung 37 in der Anschlagplatte 35 gleiten und den eigentlichen Setzvorgang auslösen.

In der Figur 13 sind zusätzlich die Kurvenflächen 26',26",26‴,26ʺʺ des Kurventrägers 21 eingezeichnet, welche die Bewegung des ersten Läufers 17 und des zweiten Läufers 18 über den Steuerbereich vorgeben.

In Figur 14 ist das Steuerelement 19 bereits über den ersten Teil des Steuerbereiches verschoben (in Pfeilrichtung). Dies hat mittels der Fläche 26ʺʺ zu einem simultan-gleichgerichteten Verschieben der beiden Läufer 17, 18 und damit zu einem Setzen der Verweilkanüle 16 mithilfe der Einstechkanüle 15 geführt.

Es versteht sich von selbst, dass es während des Verschiebens des Steuerelementes 19 zu einer Expansion der Schraubenfeder 10 kommt. Aus zeichnungstechnischen Gründen ist diese in den Figuren 13 bis 16 allerdings immer im komprimierten Zustand gezeigt.

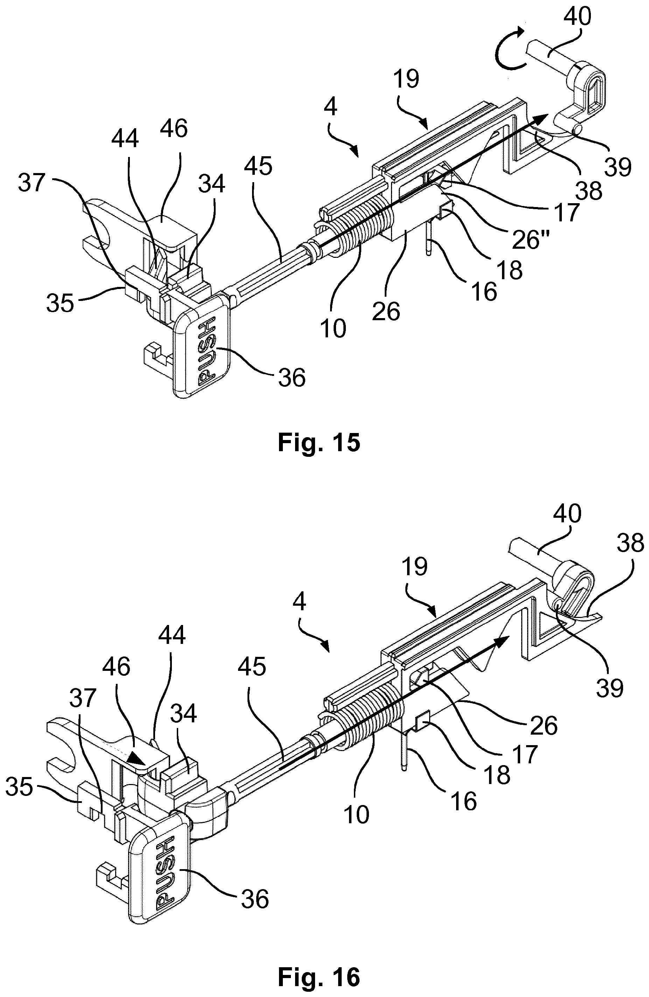

Die Figur 15 zeigt das Verschieben des Steuerelementes 19 über einen zweiten Teil des Steuerbereiches. Dabei wird zum einen ein Blockieren des als Halteblech ausgeführten zweiten Läufers 18, und damit ein Halten der Verweilkanüle 16 in der Verweilposition, lediglich durch Einwirkung, namentlich durch Herabdrücken, durch das Steuerelement 19 mit der Kurvenfläche 26 erzielt. Zum anderen wird mittels der Kurvenfläche 26'' ein Zurückziehen der Einstechkanüle 15 aus dem distalen Endbereich der Verweilkanüle 16 erzielt.

Das Steuerelement 19 weist eine zusätzliche Kurvenfläche 38 auf, die im zweiten Teil des Steuerbereiches auf das Hebelelement 39 wirkt. Damit ist das Hebelelement 39 von der in Figur 15 gezeigten ersten Position in eine in Figur 16 abgebildete zweite Position bewegbar, wodurch es zu einer Rotation einer Drehachse 40 kommt. Das Steuerelement 19 kann damit weitere Funktionen einer entsprechenden Vorrichtung, beispielsweise eines Dosiergerätes kontrollieren. So kann eine Aktivierung einer Ventil- oder Fördervorrichtung, um ein Fluid zur Injektionsvorrichtung zu leiten, realisiert werden. Beispielsweise kann eine Ventilvorrichtung so umgeschaltet werden, dass die Flüssigkeit durch ein Transfermittel 5 in die gewünschte Richtung, namentlich vom ersten Behälter 2 hin zur Injektionsvorrichtung, gelenkt wird.

Im vorliegenden Ausführungsbeispiel weist die Injektionsvorrichtung zusätzlich noch eine weitere Kurvenfläche 44 auf, die nicht direkt am Steuerelement 19 angebracht ist. Die besagte Kurvenfläche 44 ist einstückig mit dem Anschlagelement 34 ausgebildet und über eine Stange 45 mit dem Steuerelement 19 verbunden. Wenn der Push Button 36 in die in Figur 13 eingezeichnete Pfeilrichtung bewegt wird, wird das Element 34 so verschoben, dass es durch die Aussparung 37 passt. Dadurch wird die Verschiebung des Steuerelements 19 entlang dem in Figur 14 eingezeichneten Pfeil ausgelöst. Während das Anschlagelement 34 zusammen mit dem Steuerelement in die eingezeichnete Pfeilrichtung gleitet, wirkt es auf den Schieber 46 und verschiebt diesen. Die Bewegung des Schiebers 46 ist in den Figuren 15 und 16 erkennbar. Die Schiebebewegung kommt dadurch zustande, dass die Kurvenfläche 44 durch eine Aussparung in den Schieber 46 eingreift. Der Schieber 46 wird dadurch entlang einer Verschiebungsrichtung senkrecht zur Verschiebungsrichtung des Steuerelementes 19 transferiert (gepunkteter Pfeil). Alternativ kann der Schieber in einer Schrägrichtung verschoben werden, die einen Winkel zwischen dem gepunkteten Pfeil und dem durchgängigen Pfeil (Figur 16) beschreibt.

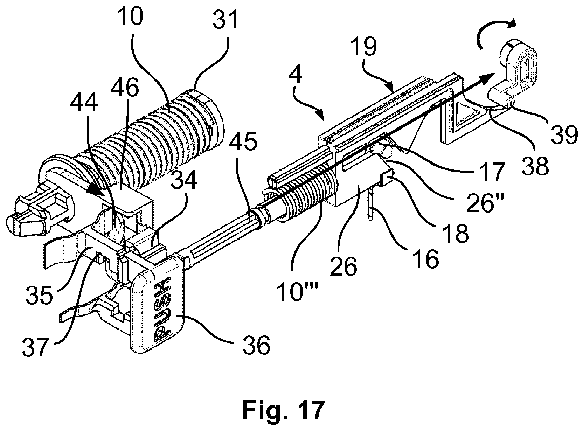

Die Figur 17 zeigt nochmal den in Figur 15 gezeigten Zustand. Zur Verdeutlichung der Funktion des Schiebers wurde jedoch dessen Interaktion mit einer Kolbenstange 31 gezeichnet. Die Bewegung des Schiebers hin zum Steuerelement ist durch den gepunkteten Pfeil dargestellt. Die Bewegung der Kolbenstange 31, die auf den Kolben 9 im ersten Behälter 2 einwirkt, ist als durchgängiger Pfeil dargestellt. Durch das Lösen des Federelements 10, welches zuvor durch den Schieber 46 in gespannter Position festgehalten wurde, wird die Schiebebewegung der Kolbenstange 31 und damit die Injektion ausgelöst. Das Verschieben des Schiebers kann gleichzeitig mit dem Umschalten des Ventils durch Hebel 40 erfolgen oder dem Umschalten des Ventils zeitlich nachgelagert sein, wobei der Zeitverzug zwischen den Auslösevorgängen sehr kurz ist.

## Patentansprüche

1. Vorrichtung (1) zur Abgabe einer Substanz, insbesondere an einen Patienten, umfassend einen die Substanz enthaltenden zylinderförmigen ersten Behälter (2), einen eine Flüssigkeit enthaltenden zweiten Behälter (3), eine Injektionsvorrichtung (4) sowie Transfermittel (5) zum Transferieren der Flüssigkeit vom zweiten (3) zum ersten Behälter (2) und vom ersten Behälter (2) zur Injektionsvorrichtung (4), wobei der erste Behälter (2) an einem ersten Ende (6) ein Anschlusselement (7) und einen zwischen dem ersten und einem zweiten Ende (8) verschiebbaren Kolben (9) aufweist, wobei durch Verschieben des Kolbens (9) gegen das erste Ende (6) des ersten Behälters (2) eine darin enthaltene Flüssigkeit über die Injektionsvorrichtung (4) abgebbar ist,
**dadurch gekennzeichnet, dass**
die Injektionsvorrichtung (4) eine Einstechkanüle (15) und eine Verweilkanüle (16) umfasst, wobei in einer Ausgangsposition ein distaler Endbereich der Einstechkanüle (15) koaxial im Inneren der Verweilkanüle (16) verläuft und wobei der erste Behälter (2) über eine Ventilanordnung (5 ) wahlweise mit dem zweiten Behälter (3) oder der Injektionsvorrichtung (4) in Fluidkommunikation bringbar ist.

2. Vorrichtung nach Anspruch 1, wobei die Injektionsvorrichtung (4) einen ersten und einen zweiten verschiebbar gelagerten Läufer (17, 18) umfasst, wobei der erste Läufer mit der Einstechkanüle (15) und der zweite Läufer mit der Verweilkanüle (16) verbunden ist und wobei die Injektionsvorrichtung ferner ein über einen vordefinierten Steuerbereich bewegbares Steuerelement (19) umfasst, das zum Verschieben des ersten Läufers (17) und des zweiten Läufers (18) mit diesen in Wirkverbindung bringbar ist.

3. Vorrichtung nach Anspruch 2, wobei das Steuerelement (19) über ein Federelement (10‴) vorgespannt ist.

4. Vorrichtung (1) nach einem der Ansprüche 1 bis 3, wobei der zweite Behälter (3) ebenfalls zylinderförmig mit einem Anschlusselement (7') an einem ersten Ende (6') sowie einem zwischen dem ersten und einem zweiten Ende (8') verschiebbaren Kolben (9') ausgestaltet ist.

5. Vorrichtung (1) nach einem der Ansprüche 1 bis 4, wobei der Kolben (9) des ersten Behälters (2), und gegebenenfalls auch der Kolben (9') des zweiten Behälters (3), durch Verschiebungsmittel verschiebbar ist/sind.

6. Vorrichtung (1) nach Anspruch 5, wobei der Kolben (9) des ersten Behälters (2), und gegebenenfalls auch der Kolben (9') des zweiten Behälters (3), durch jeweils ein Federelement (10, 10') vorgespannt und damit verschiebbar ist/sind.

7. Vorrichtung (1) nach Anspruch 6, wobei der Kolben (9) des ersten Behälters (2), und gegebenenfalls auch der Kolben (9') des zweiten Behälters (3), über Rückhaltemittel (11, 11') gegen die Vorspannung zurückgehalten ist/sind.

8. Vorrichtung (1) nach einem der Ansprüche 1 bis 7, wobei das Anschlusselement (6) des ersten Behälters (2), und gegebenenfalls auch dasjenige des zweiten Behälters (3), als Septum (12, 12') ausgebildet ist/sind.

9. Vorrichtung (1) nach Anspruch 8, wobei das Septum (12) des ersten Behälters (2), und gegebenenfalls auch dasjenige (12') des zweiten Behälters (3), mit Penetrationsmitteln (13, 13'), insbesondre durch Verschieben des ersten Behälters (2), und gegebenenfalls auch des zweiten Behälters (3), gegen das/die Penetrationsmittel (13, 13'), penetrierbar ist/sind.

10. Vorrichtung (1) nach einem der Ansprüche 1 bis 9, wobei der erste Behälter (2) über das Anschlusselement (6) mit dem zweiten Behälter (3) und der Injektionsvorrichtung (4) in Fluidkommunikation bringbar ist.

11. Vorrichtung (1) nach Anspruch 7 und einem der Ansprüche 8 bis 10, wobei die Injektionsvorrichtung (4), und auch die Verschiebungsmittel, über Aktivierungsmittel aktivierbar sind, wobei die Aktivierungsmittel zum Lösen der Rückhaltemittel (11, 11') ausgelegt sind.

12. Vorrichtung (1) nach Anspruch 11, wobei die Injektionsvorrichtung (4) und ein Hebel (39) zum Umschalten der Ventilvorrichtung über dasselbe Aktivierungsmittel aktivierbar sind.

13. Vorrichtung nach Anspruch 2 und einem der Ansprüche 3 bis 12, wobei das Steuerelement als verschiebbarer Kurventräger ausgebildet ist.

14. Vorrichtung nach Anspruch 2 und einem der Ansprüche 3 bis 12, wobei das Steuerelement einen linear verschiebbaren Schlitten umfasst, an dem wenigstens ein Kurventräger ausgebildet ist.

## Claims

1. Device (1) for dispensing a substance, in particular to a patient, comprising a cylindrical first container (2) containing the substance, a second container (3) containing a liquid, an injection device (4), and transfer means (5) for transferring the liquid from the second container (3) to the first container (2) and from the first container (2) to the injection device (4), wherein the first container (2) has a connecting element (7) at a first end (6) and a piston (9) which can be displaced between the first end and a second end (8), wherein a liquid contained in said first container can be dispensed via the injection device (4) by displacing the piston (9) toward the first end (6) of the first container (2),
**characterized in that**
the injection device (4) comprises a puncture cannula (15) and an indwelling cannula (16), wherein in a starting position a distal end region of the puncture cannula (15) extends coaxially in the interior of the indwelling cannula (16) and wherein the first container (2) can be brought into fluid communication with either the second container (3) or the injection device (4) via a valve arrangement (5).

2. Device according to claim 1, wherein the injection device (4) comprises a first and a second displaceably mounted runner (17, 18), wherein the first runner is connected to the puncture cannula (15) and the second runner is connected to the indwelling cannula (16), and wherein the injection device further comprises a control element (19) which can be moved over a predefined control region and which can be brought into operative connection with the first runner (17) and the second runner (18) in order to displace them.

3. Device according to claim 2, wherein the control element (19) is preloaded via a spring element (10‴).

4. Device (1) according to any of claims 1 to 3, wherein the second container (3) is also cylindrical and has a connecting element (7') at a first end (6') and a piston (9') which can be displaced between the first end and a second end (8').

5. Device (1) according to any of claims 1 to 4, wherein the piston (9) of the first container (2), and where applicable also the piston (9') of the second container (3), can be displaced by displacement means.

6. Device (1) according to claim 5, wherein the piston (9) of the first container (2), and where applicable also the piston (9') of the second container (3), is/are preloaded by a particular spring element (10, 10') and can be displaced thereby.

7. Device (1) according to claim 6, wherein the piston (9) of the first container (2), and where applicable also the piston (9') of the second container (3), is/are retained via retaining means (11, 11') against the preload.

8. Device (1) according to any of claims 1 to 7, wherein the connecting element (6) of the first container (2), and where applicable also that of the second container (3), is/are in the form of a septum (12, 12').

9. Device (1) according to claim 8, wherein the septum (12) of the first container (2), and where applicable also that (12') of the second container (3), can be penetrated using penetration means (13, 13'), in particular by displacing the first container (2), and where applicable also the second container (3), against the penetration means (13, 13').

10. Device (1) according to any of claims 1 to 9, wherein the first container (2) can be brought into fluid communication with the second container (3) and the injection device (4) via the connecting element (6).

11. Device (1) according to claim 7 and any of claims 8 to 10, wherein the injection device (4), and also the displacement means, can be activated via activation means, wherein the activation means are designed to release the retaining means (11, 11').

12. Device (1) according to claim 11, wherein the injection device (4) and a lever (39) for switching the valve device can be activated via the same activation means.

13. Device according to claim 2 and any of claims 3 to 12, wherein the control element is in the form of a displaceable cam carrier.

14. Device according to claim 2 and any of claims 3 to 12, wherein the control element comprises a linearly displaceable carriage on which at least one cam carrier is formed.

## Revendications

1. Dispositif (1) permettant d'administrer une substance, en particulier à un patient, comprenant un premier récipient (2) de forme cylindrique contenant la substance, un second récipient (3) contenant un liquide, un dispositif d'injection (4) ainsi que des moyens de transfert (5) pour le transfert du liquide du second (3) au premier récipient (2) et du premier récipient (2) au dispositif d'injection (4), dans lequel le premier récipient (2) présente à une première extrémité (6) un élément de raccordement (7) et un piston (9) pouvant coulisser entre la première et une seconde extrémité (8), dans lequel un liquide contenu dans le premier récipient peut être administré par l'intermédiaire du dispositif d'injection (4) en faisant coulisser le piston (9) contre la première extrémité (6) du premier récipient (2),
**caractérisé en ce que**
le dispositif d'injection (4) comprend une canule de perçage (15) et une canule à demeure (16), dans lequel, dans une position initiale, une zone d'extrémité distale de la canule de perçage (15) s'étend de manière coaxiale à l'intérieur de la canule à demeure (16) et dans lequel le premier récipient (2) peut être amené en communication fluidique, au choix avec le second récipient (3) ou le dispositif d'injection (4), par l'intermédiaire d'un agencement formant soupape (5).

2. Dispositif selon la revendication 1, dans lequel le dispositif d'injection (4) comprend un premier et un second curseur (17, 18) montés de manière à pouvoir coulisser, dans lequel le premier curseur est relié à la canule de perçage (15) et le second curseur est relié à la canule à demeure (16), et dans lequel le dispositif d'injection comprend en outre un élément de commande (19) pouvant être déplacé sur une plage de commande prédéfinie et pouvant être amené en liaison active avec le premier curseur (17) et le second curseur (18) pour les faire coulisser.

3. Dispositif selon la revendication 2, dans lequel l'élément de commande (19) est précontraint par l'intermédiaire d'un élément formant ressort (10‴).

4. Dispositif (1) selon l'une des revendications 1 à 3, dans lequel le second récipient (3) est également conçu sous forme cylindrique avec un élément de raccordement (7') à une première extrémité (6') ainsi qu'un piston (9') pouvant coulisser entre la première et une seconde extrémité (8').

5. Dispositif (1) selon l'une des revendications 1 à 4, dans lequel le piston (9) du premier récipient (2), et éventuellement également le piston (9') du second récipient (3), peuvent coulisser par l'intermédiaire de moyens de coulissement.

6. Dispositif (1) selon la revendication 5, dans lequel le piston (9) du premier récipient (2), et éventuellement également le piston (9') du second récipient (3), sont précontraints par respectivement un élément formant ressort (10, 10') et peuvent ainsi coulisser.

7. Dispositif (1) selon la revendication 6, dans lequel le piston (9) du premier récipient (2), et éventuellement également le piston (9') du second récipient (3), sont retenus à l'encontre de la précontrainte par l'intermédiaire de moyens de retenue (11, 11').

8. Dispositif (1) selon l'une des revendications 1 à 7, dans lequel l'élément de raccordement (6) du premier récipient (2), et éventuellement également celui du second récipient (3), sont réalisés sous forme de septums (12, 12').

9. Dispositif (1) selon la revendication 8, dans lequel le septum (12) du premier récipient (2), et éventuellement également celui (12') du second récipient (3), peuvent être pénétrés par des moyens de pénétration (13, 13'), en particulier en faisant coulisser le premier récipient (2), et éventuellement également le second récipient (3), contre le ou les moyens de pénétration (13, 13').

10. Dispositif (1) selon l'une des revendications 1 à 9, dans lequel le premier récipient (2) peut être amené en communication fluidique avec le second récipient (3) et le dispositif d'injection (4) par l'intermédiaire de l'élément de raccordement (6).

11. Dispositif (1) selon la revendication 7 et l'une des revendications 8 à 10, dans lequel le dispositif d'injection (4), et également les moyens de coulissement, peuvent être activés par l'intermédiaire de moyens d'activation, dans lequel les moyens d'activation sont adaptés pour libérer les moyens de retenue (11, 11').

12. Dispositif (1) selon la revendication 11, dans lequel le dispositif d'injection (4) et un levier (39) pour la commutation du dispositif formant soupape peuvent être activés par l'intermédiaire desdits moyens d'activation.

13. Dispositif selon la revendication 2 et l'une des revendications 3 à 12, dans lequel l'élément de commande est réalisé comme un support pour cames pouvant coulisser.

14. Dispositif selon la revendication 2 et l'une des revendications 3 à 12, dans lequel l'élément de commande comprend un chariot pouvant coulisser linéairement, sur lequel est réalisé au moins un support pour cames.
